# EUROPÄISCHE PATENTSCHRIFT

(11) **EP 2 581 890 B1**
(45) Veröffentlichungstag und Bekanntmachung des Hinweises auf die Patenterteilung: **26.02.2014**
(21) Anmeldenummer: 11185328.9
(22) Anmeldetag: 14.10.2011
(51) Int. Cl.: G01N 33/00, G08B 17/117, G08B 29/18, G08B 29/24, G01N 27/414

(54) **Verfahren zur Erhöhung der Fehlalarmsicherheit eines Brandmelders**
Method for decreasing the possibility of a fire alarm producing a false alarm
Procédé d'augmentation de la sécurité contre les fausses alarmes d'une alerte incendie

(43) Veröffentlichungstag der Anmeldung: 17.04.2013
(73) Patentinhaber: Siemens Aktiengesellschaft, 80333 München (DE)
(72) Erfinder: Gutmacher, Daniel, 79102 Freiburg (DE); Hoefer, Ulrich, Dr., 6300 Zug (CH)

(56) Entgegenhaltungen:
- EP-A2- 2 336 762
- WO-A1-2010/112476
- DE-A1-102008 048 715

## Beschreibung

Die Erfindung betrifft ein Verfahren zur Erhöhung der Fehlalarmsicherheit eines Brandmelders. Darüber hinaus betrifft die Erfindung einen Brandmelder.

Gassensitive Feldeffekttransistoren können verwendet werden, um Brandgase zu detektieren. Es ist bekannt, solche Feldeffekttransistoren in Gassensoren für Brandmelder zu verwenden.

Voraussetzung für eine zuverlässige Branderkennung sowie hohe Störsicherheit, insbesondere die Unterdrückung von Fehlalarmen, sind u. a. reproduzierbare Signale sowie die Unempfindlichkeit der Sensoren gegenüber Störeinflüssen wie Luftfeuchteschwankungen, Temperaturschwankungen, Licht und das Auftreten von anderen Gasen (Querempfindlichkeiten).

Feldeffekttransistoren-basierte Gassensoren können eine starke Abhängigkeit gegenüber hohen Luftfeuchten, schnellen Luftfeuchteänderungen bzw. Taupunktüberschreitung zeigen. Grund hierfür sind Kondensationseffekte an mikroskopisch kleinen, als Kondensationskeim dienenden Verunreinigungen bzw. produktionstechnisch bedingten Unebenheiten im Gate-Spalt. Durch die Feuchte können sich ionenleitende Pfade zwischen Gate und Gatepassivierung bzw. zwischen Gate und anderen leitenden Bestandteilen des Transistors ausbilden und es kann in Folge zu chaotischen, nicht reproduzierbaren Signalverläufen kommen. Werden diese nicht als Störung durch Feuchte erkannt, so kann dies von den Auswertealgorithmen als Brand identifiziert werden und es kommt zu einem Fehlalarm. Um derartige Störeinflüsse zu erkennen, mussten bisher zusätzliche Feucht- bzw. Taupunktfühler eingesetzt werden.

Aus der Offenlegungsschrift DE 10 2008 048 715 A1 ist ein Verfahren zur Bestimmung der Feuchte in einem Gas mit Hilfe eines Feldeffekttransistor-basierten Gassensor bekannt. Bei diesem Verfahren wird an einem Gate des Feldeffekttransistors eine zusätzliche Potentialänderung eingeprägt, die eine Änderung des Sensorsignals zur Folge hat. Anschließend wird eine Größe der sich ergebenden Änderung des Sensorsignals bezogen auf die zusätzliche Potentialänderung ausgewertet. Dieser Größe wird anhand einer zuvor ermittelten Sensorkennlinie eine eindeutige relative Feuchte zugeordnet.

Eine Aufgabe der vorliegenden Erfindung ist es, die Zuverlässigkeit eines Brandmelders zu erhöhen. Diese Aufgabe wird durch ein Verfahren nach Anspruch 1 bzw. einem Brandmelder nach Anspruch 9 gelöst.

Das erfindungsgemäße Verfahren dient zur Erhöhung der Fehlalarmsicherheit eines Brandmelders, der einen Brandgassensor aufweist, welcher einen gassensitiven Feldeffekttransistor umfasst. Dabei wird an einem Gate des Feldeffekttransistors eine zusätzliche Potentialänderung eingeprägt, die eine Änderung des Sensorsignals zur Folge hat. Wenigstens eine feuchteabhängige Größe dieser Signaländerung wird ausgewertet und der Brandmelder wird zur Vermeidung eines feuchteverursachten Fehlalarms in einen Störungsmodus versetzt, wenn ein bestimmtes Auswerteergebnis vorliegt.

Der erfindungsgemäße Brandmelder weist einen Brandgassensor auf, der einen gassensitiven Feldeffekttransistor umfasst. An einem Gate des Feldeffekttransistors ist mit Hilfe einer Spannungsquelle eine zusätzliche Potentialänderung einprägbar, die eine Änderung des Sensorsignals zur Folge hat. Der Brandmelder weist eine Auswerteeinrichtung auf zur Auswertung wenigstens einer feuchteabhängigen Größe einer solchen Sensorsignaländerung. Darüber hinaus weist der Brandmelder eine Kontrolleinrichtung auf, die dazu ausgebildet ist, den Brandmelder zur Vermeidung eines feuchteverursachten Fehlalarms in einen Störungsmodus zu versetzen, wenn ein bestimmtes Auswerteergebnis vorliegt.

Die im Folgenden im Zusammenhang mit dem Verfahren erläuterten Vorteile und Ausgestaltungen gelten sinngemäß auch für den erfindungsgemäßen Brandmelder und umgekehrt.

Die Erfindung greift das aus dem Stand der Technik bekannte Vorgehen auf, wonach an einem Gate des Feldeffekttransistors eine zusätzliche Potentialänderung eingeprägt wird, die eine Änderung des Sensorsignals zur Folge hat. Der Gassensor wird jedoch nicht zur Feuchtemessung verwendet. Eine Bestimmung der Feuchte findet nicht statt. Stattdessen erfolgt eine Auswertung der Sensorsignaländerung im Hinblick auf die Existenz feuchteinduzierter Signalstörungen. Die sich aus der zusätzlich eingeprägten Potentialänderung ergebende Sensorsignaländerung wird mit anderen Worten dahingehend untersucht, ob sie Rückschlüsse auf das Vorliegen eines Störeinflusses, nämlich der Feuchte, zulässt. Nachdem die Sensorsignaländerung ausgewertet und auf das Vorliegen feuchteabhängiger Signalstörungen hin untersucht ist, wird auf der Grundlage des Auswerteergebnisses über eine Änderung des Betriebsmodus des Brandmelders entschieden. Sollten sich aus der Änderung des Sensorsignals Anhaltspunkte dafür ergeben, dass der Brandgassensor aufgrund von z. B. Kondensationseffekten ein falsches Sensorsignal liefert, wird eine Störung angenommen und ein Alarm unterdrückt.

Mit der vorliegenden Erfindung wird die Brandmelderzuverlässigkeit deutlich erhöht. Klassische Fehlalarmszenarien, beispielsweise verursacht durch Wasserdampf bei optischen Rauchmeldern, werden unterdrückt. Der Einsatz zusätzlicher Feuchte- bzw. Taupunktfühler ist nicht mehr notwendig, wodurch die Herstellungskosten gesenkt werden können.

Vorteilhafte Ausführungen sind in den Unteransprüchen angegeben.

Die Erfindung macht sich die Erkenntnis zunutze, dass sich der Signalverlauf des Sensorsignals als Antwort auf die zusätzliche Potentialänderung verändert. Insbesondere lässt sich, was bisher unberücksichtigt bliebt, ein feuchteabhängiger Unterschied zwischen zwei während der zusätzlichen Potentialänderung erfassten Sensorsignalen feststellen. Entsprechend wird in einem bevorzugten Ausführungsbeispiel der Erfindung eine Signaldifferenz zwischen einem ersten und einem zweiten Zeitpunkt während der zusätzlichen Potentialänderung ausgewertet. Insbesondere die Auswertung des zeitlichen Verlaufs einer solchen Signaldifferenz hat sich als besonders vorteilhaft erwiesen, da sich herausgestellt hat, dass insbesondere die Änderung des Signaldifferenzwertes und die Dauer der Signaländerung eine Aussage über das Vorliegen einer Taupunktüberschreitung zulässt.

Zusätzlich oder alternativ zu einer Auswertung der Signaldifferenz zwischen zwei Zeitpunkten während der zusätzlich Potentialänderung wird in einem weiteren Ausführungsbeispiel der Verlauf des Sensorsignals zwischen einem ersten und einem zweiten Zeitpunkt während der zusätzlichen Potentialänderung ausgewertet. Sowohl die Signalform, als auch die Größe der Signaländerung können Rückschlüsse auf das Vorliegen erratischer Signale geben.

In einem einfachen Fall wird dabei der zu untersuchende Zeitabschnitt derart gewählt, dass der erste Zeitpunkt zu Beginn der zusätzlichen Potentialänderung und der zweite Zeitpunkt am Ende der zusätzlichen Potentialänderung liegt. Mit anderen Worten wird die Signaldifferenz bzw. der Signalverlauf vorzugsweise während des gesamten Zeitraums der zusätzlichen Potentialänderung betrachtet. Je nach Art des verwendeten Feldeffekttransistors können feuchteabhängige Signaländerungen aber auch verstärkt am Anfang, während und/oder am Ende der zusätzlichen Potentialänderung auftreten, so dass es ausreichend sein kann, nur einen bestimmten Zeitabschnitt, beispielsweise den Beginn der zusätzlichen Potentialänderung oder das Ende der zusätzlichen Potentialänderung oder aber einen begrenzten Zeitabschnitt während der zusätzlichen Potentialänderung zu betrachten.

In einem einfachen Fall, der sich zugleich als für eine zuverlässige Fehlalarmvermeidung besonders geeignet erwiesen hat, wird die zusätzliche Potentialänderung in Form eines Spannungsimpulses eingeprägt. Die Verwendung eines Pulsbetriebs stellt die Aufrechterhaltung der ordnungsgemäßen Funktion des Brandmelders sicher, da zwischen dem Anlegen der Pulsspannung auch weiterhin eine Brandüberwachung möglich ist. Vorzugsweise erfolgt dabei das Einprägen einer Rechteckspannung. Eine derartige zusätzliche Potentialänderung kann einerseits auf besonders einfache Art und Weise erzeugt werden. Andererseits hat sich diese Signalform als besonders geeignet erwiesen für die Auswertung feuchteinduzierter Signaländerungen.

Das Auftreten einer Kondensation lässt sich besonders sicher dadurch erkennen, dass sich das Sensorsignal besonders schnell ändert, obwohl der Wert des eingeprägten Potentials konstant ist. Ein weiteres Anzeichen dafür, dass es sich um eine feuchteinduzierte Störung handelt, ist die Dauer der Abweichung des Signaldifferenzwertes vom Normalwert. In einer bevorzugten Ausführungsform wird daher auch die Abweichungsdauer ausgewertet.

Alternativ zu einer Rechteckspannung kann auch ein anders geformter Spannungsimpuls eingeprägt werden. Andere Signalformen, beispielsweise Sinus- oder Rechtecksignale, sind prinzipiell möglich. Jedoch ist ein sich aufgrund einer eingeprägten Rechteckspannung änderndes Sensorsignal besonders einfach und eindeutig interpretierbar.

Vorteilhafterweise erfolgt ein wiederholtes Einprägen der zusätzlichen Potentialänderung, vorzugsweise in regelmäßigen Abständen. Dies ermöglicht eine besonders hohe Störsicherheit, da es eine mehrmalige, vorzugsweise regelmäßige Auswertung der Signaländerungen ermöglicht. Sind die zeitlichen Abstände, in denen das Einprägen der zusätzlichen Potentialänderung und das Auswerten des Sensorsignale erfolgt, gering, ist eine beim Einsatz der Gassensoren in Brandmeldern besonders vorteilhafte fortlaufende Kontrolle der Feuchte als Störgröße möglich.

Zur Durchführung des erfindungsgemäßen Verfahrens weist der Brandmelder eine die beschriebene Auswertung der Sensorsignale durchführende Auswerteeinrichtung auf. Diese Auswerteeinrichtung umfasst vorzugsweise eine Recheneinheit mit einem Prozessor, auf dem ein Computerprogrammprodukt zur Verwirklichung der beschriebenen Verfahrensfunktionen ausgeführt wird. Die Recheneinheit kann jedoch auch als reines Hardwaremodul ausgeführt sein.

Die Spannungsquelle zum Einprägen der zusätzlichen Potentialänderung kann als interne Spannungsquelle des Brandgassensors bzw. des Brandmelders vorgesehen sein. Es ist jedoch ebenso möglich, zum Einprägen der zusätzlichen Potentialänderung eine externe Spannungsquelle zu verwenden.

Die Erfindung wird bei Gefahrenmeldern, insbesondere Brandmeldern eingesetzt, die einen Gassensor mit gassensitivem Feldeffekttransistor verwenden. Bei dem gassensitiven Feldeffekttransistor handelt es sich insbesondere um einen Suspended Gate-Feldeffekttransistor (SGFET).

Ein SGFET ist ein gassensitiver, ein Gate aufweisender Feldeffekttransistor mit einem Luftspalt zwischen einem passivierten, zwischen einem Drain und einer Source erzeugten Kanal und einer Schicht eines Sensormaterials, das ein Bestandteil des Gates ist. Eine Verwendung eines SGFET ist eine Adsorption von Molekülen eines zu detektierenden Gases auf der Oberfläche des Sensormaterials und dabei erfolgendes Erzeugen einer Dipolschicht und eines elektrischen Potentials, das über den Luftspalt die Kanalleitfähigkeit und damit einen Source-Drain-Strom beeinflusst, der durch einen elektrischen Widerstand fließt und damit eine Spannung erzeugt, deren Änderung das Sensorsignal ist.

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung der Ausführungsbeispiele, die im Zusammenhang mit den Zeichnungen näher erläutert werden. Dabei zeigen:
- FIG 1: eine schematische Darstellung eines Brandmelders mit einem SGFET,
- FIG 2: eine vereinfachte Schnittdarstellung eines SGFET,
- FIG 3: ein Sensorsignal bei zusätzlichen Potentialänderungen unter normalen Bedingungen,
- FIG 4: ein Sensorsignal bei zusätzlichen Potentialänderungen bei Kondensation,
- FIG 5: ein Sensorsignal während typischer Gasbeaufschlagungen und hohen Feuchten sowie die dazugehörigen Signaldifferenzen.

Sämtliche Figuren zeigen die Erfindung lediglich schematisch und mit ihren wesentlichen Bestandteilen. Gleiche Bezugszeichen entsprechen dabei Elementen gleicher oder vergleichbarer Funktion.

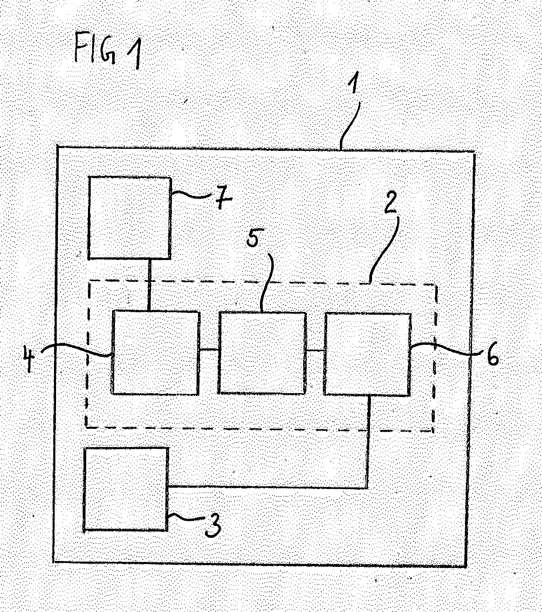

FIG 1 zeigt schematisch einen Brandmelder 1 mit einem Gassensor 2 und einer mit dem Gassensor 2 verbundenen Meldeeinheit 3, die u.a. zur Übertragung eines Alarmsignals an eine mit dem Brandmelder 1 verbundene Meldezentrale (nicht abgebildet) ausgebildet ist. Der Gassensor 2 umfasst einen SGFET (Suspended Gate Feldeffekttransistor) 4, eine damit verbundene Auswerteeinrichtung 5 und eine mit der Auswerteeinrichtung 5 verbundene Kontrolleinrichtung 6. Außerdem umfasst der Brandmelder 1 eine Spannungsquelle 7, die mit dem SGFET 4 verbunden ist. Die Funktionen der einzelnen Komponenten werden nachfolgend näher erläutert.

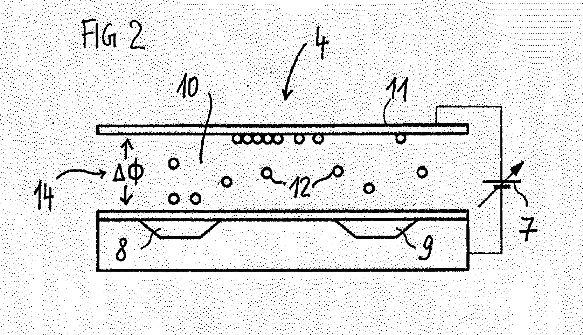

Der Aufbau eines SGFET 4, wie er zur Anwendung als Gassensor 2 in einem Brandmelder 1 erfindungsgemäß verwendet werden kann, ist in FIG 2 stark vereinfacht dargestellt. Der grundsätzliche Aufbau und die Betriebsweise eines SGFET 4 sind aus dem Stand der Technik bekannt. Der SGFET weist eine Source-Elektrode 8 und eine Drain-Elektrode 9 auf. Voneinander getrennt durch einen Luftspalt 10 ist eine gassensitive Schicht 11 angeordnet ("Suspended Gate"). Durch Adsorption des den Luftspalt 10 durchströmenden, zu detektierenden Gases 12 an der gassensitiven Schicht 11 wird eine erste Potentialänderung hervorgerufen. Das Sensorsignal ist im einfachsten Fall ein Source-Drain-Strom. In dem hier beschriebenen Ausführungsbeispiel ist das Sensorsignal eine durch den Source-Drain-Strom an einem elektrischen Widerstand erzeugte Spannung 13. Entsprechend der üblichen Arbeitsweise des Brandmelders 1 bewirkt eine durch Gasadsorption hervorgerufene erste Potentialänderung eine Änderung des Sensorsignals 13, wodurch das Vorhandensein eines Gases erkannt und erforderlichenfalls ein Alarm ausgelöst wird.

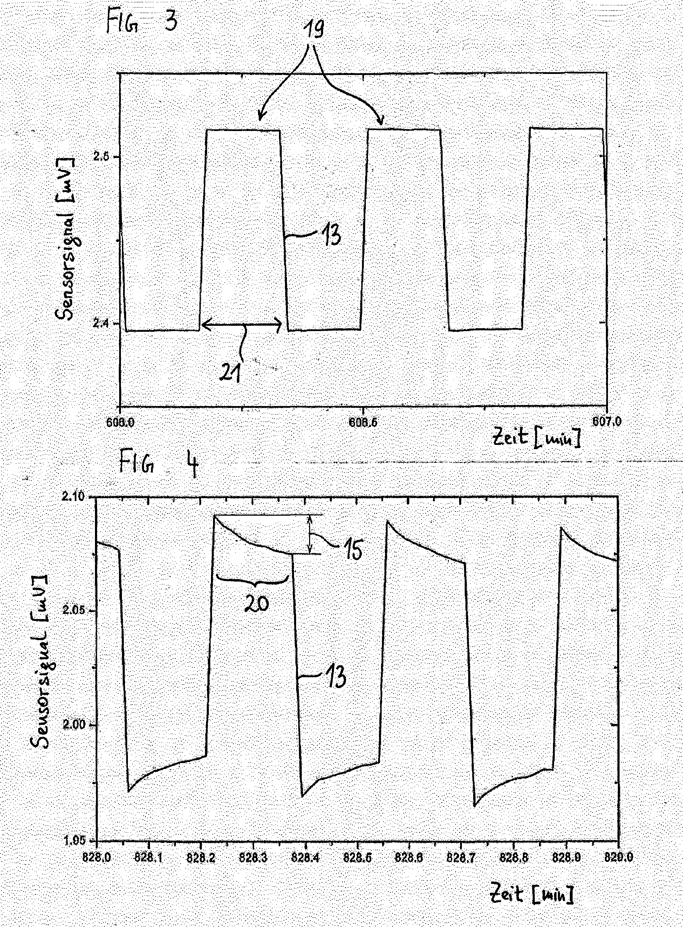

Erfindungsgemäß wird zu dieser ersten Potentialänderung eine zusätzliche Potentialänderung an dem Gate des SGFET 4 eingeprägt. Dies erfolgt durch Anlegen einer elektrischen Spannung 14 mit Hilfe der Spannungsquelle 7. Dabei handelt es sich um nach Art von Spannungspulsen in regelmäßigen Abständen angelegte Rechteckspannungen. Im Verhältnis zu der Pulsdauer sind die Impulsflanken dabei vorzugsweise steil. Durch die zusätzliche Potentialänderung kommt es zu einer Beeinflussung der Gatespannung durch die Änderung der Austrittsarbeit an der sensitiven Schicht 11. Dies bewirkt eine Änderung des Sensorsignals 13. Auf das Anlegen dieser Spannungspulse reagiert der SGFET 4 unter normalen Bedingungen mit einem Sensorsignal 13, welches entsprechend den eingeprägten Spannungsimpulsen ebenfalls rechteckförmige Spannungspulse 19 aufweist. Ein solcher Verlauf des Sensorsignals 13 als Antwort auf die zusätzlichen Potentialänderungen ist in FIG 3 dargestellt. Normalbedingungen sind in diesem Beispiel Luft mit einer relativen Feuchte von 50% bei einer Temperatur von 25°C.

Wird nun der Gassensor 2 höheren relativen Feuchten oder schnellen Feuchteänderungen ausgesetzt, so dass es zu Kondensationseffekten kommt, so reagiert der SGFET 4 auf das Anlegen der Spannungspulse mit einem veränderten Signal 13. Beispielsweise kommt es zu einem lade-/entladeförmigen Signalverlauf, wie in FIG 4 abgebildet. Die Rechteckform der Spannungspulse 19 verändert sich deutlich, obwohl die zusätzlich angelegte Spannung 14 zwischen Beginn und Ende des eingeprägten Spannungspulses konstant ist. Das Sensorsignal 13 fällt zwischen Beginn des zusätzlich eingeprägten Spannungspulses und dessen Ende nichtlinear ab, also in einem Bereich 20, in dem die eingeprägte Spannung 14 konstant ist. Die Messbedingungen sind jetzt Luft mit einer relativen Feuchte von 90% bei einer Temperatur von 25°C. Im dargestellten Fall beträgt die Pulszeit 21 zehn Sekunden. Während dieser Pulszeit 21 tritt eine Signaländerung 15 von 17,5mV auf. Dieses Verhalten zeigt der Gassensor 2 nur bei Feuchtestörungen. Das Auftreten eines derartigen Sensorsignals 13 kann daher als sicheres Anzeichen für eine feuchteverursachte Signalstörung genutzt werden.

Da einerseits der Gasmelder während des Anlegens der Pulsspannung eine Totzeit hat, also kein Gassignal abgegriffen werden kann, andererseits aber die Pulszeit so lange sein muss, dass sich eine sinnvoll auswertbare Signaländerung, z.B. eine Spannungsdifferenz, ausbildet, haben sich vorzugsweise Pulszeiten in einem Bereich von mehreren Sekunden, insbesondere von etwa einer Sekunde bis etwa zwanzig Sekunden bewährt. Besonders vorteilhaft ist eine Pulszeit von acht bis zwölf Sekunden.

In einer Ausführungsform der Erfindung, auf die nachfolgend nicht weiter eingegangen wird, wird der Verlauf des Sensorsignals 13 während der zusätzlichen Potentialänderungen 14 ausgewertet, beispielsweise indem eine Anpassung der Sensorsignaldaten an eine Funktion erfolgt. Die Fitparameter können dann zur Beurteilung der Relevanz der Signalstörung dienen.

Im nachfolgend betrachteten, einfachsten Fall wird die Signaldifferenz 15 zwischen Beginn und Ende des Spannungspulses 14 ausgewertet. Übersteigt der Wert der Signaldifferenz 15 einen bestimmten Betrag, so wird dies als Störung gewertet. Der Brandauswertealgorithmus wird dann den Brandmelder 1 veranlassen, in Störung zu gehen.

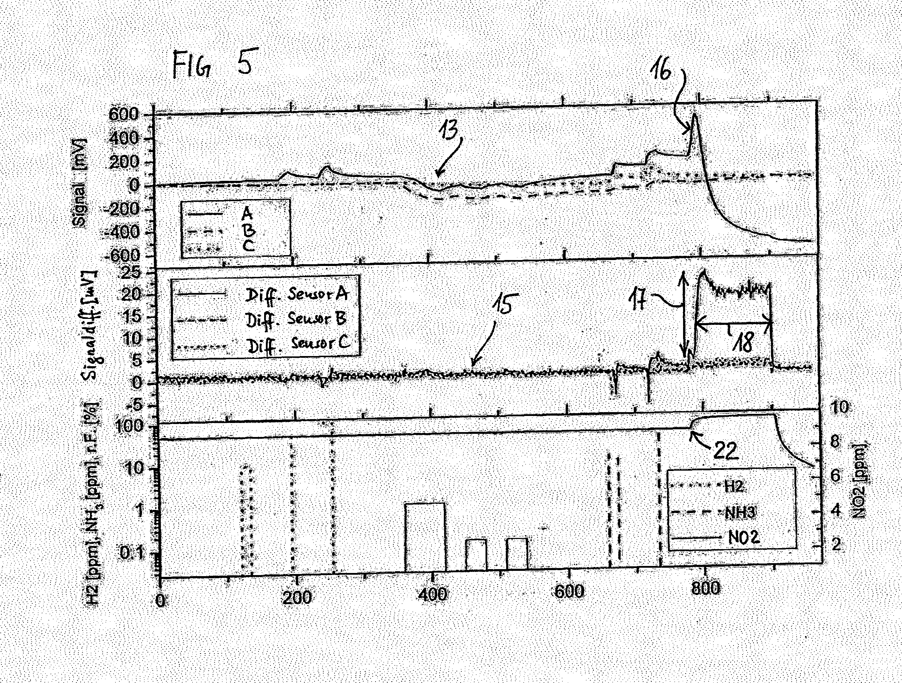

FIG 5 zeigt beispielhaft das Verhalten von drei verschiedenen Sensoren A, B, C bei verschiedenen Gasbeaufschlagungen. Dabei kann es sich um Sensoren handeln, die in einem einzigen Gasmelder 1 enthalten sind. Wie im unteren Drittel von FIG 5 dargestellt, wird zwischen 100 min und 300 min H₂ beaufschlagt. Zwischen 300 min und 600 min wird NO₂ beaufschlagt. Zwischen 600 min und 800 min wird NH₃ beaufschlagt. In dem oberen Drittel von FIG 5 sind die typischen Signalverläufe der drei Sensoren A, B, C als Antwort auf das Auftreten der verschiedenen Gase über die Zeit aufgetragen. Besonders deutlich sind die Signalanstiege des Sensors A bei H₂ im Bereich um ca. 200 min und bei NH₃ im Bereich um ca. 700 min.

Nach etwa 750 min kommt es zu einem starken Anstieg 22 der relativen Feuchte. Eine zusätzliche Gasbeaufschlagung erfolgt nicht mehr. Deutlich zu sehen ist, wie das Sensorsignal 13 des Sensors A während des Feuchteanstiegs bei t = 750 min zuerst wieder deutlich ansteigt und kurz danach, in der Plateauphase der Hochfeuchte, stark abfällt. Dieser Signalanstieg 16 könnte bei herkömmlichen Sensoren nicht von einem Gassignal unterschieden werden und würde somit zu einem Fehlalarm führen.

Wie oben beschrieben, antwortet das Sensorsignal 13 auf die angelegten Spannungsimpulse 14 mit Signaländerungen in Gestalt von entsprechenden Signalimpulsen. In dem mittleren Drittel von FIG 5 sind als feuchteabhängige Größe dieser Signaländerung die in FIG 4 dargestellten Signaldifferenzen 15 zwischen Beginn und Ende der Spannungsimpulse 14 über die Zeit aufgetragen. Bei allen drei Sensoren A, B, C zeigen sich, wie erwartet, mehr oder weniger große Schwankungen der Signaldifferenzen 15 bei der Detektion der Gase, z. B. bei etwa 250 min und im Bereich um 700 min. Diese Signalwerteschwankungen dauern jedoch nur kurze Zeit.

Deutlich unterscheiden sich die nicht gestörten, in etwa gleichbleibenden Signaldifferenzverläufe der Sensoren B und C von dem Verlauf der Signaldifferenz 15 bei Sensor A. Der sprunghafte Anstieg der Signaldifferenz 15 von Sensor A bei t = 750 min wird durch die Auswerteeinrichtung 5 erkannt und bewertet. Überschreitet die Änderung der Signaldifferenz 15 innerhalb eines bestimmten Zeitraums einen bestimmten Wert, und handelt es sich nicht nur um eine kurzzeitige Änderung, so wird angenommen, dass eine Taupunktüberschreitung und eine damit verbundene Kondensation bei Sensor A vorliegt. Im hier beschriebenen Fall bleibt der Wert der Signaldifferenz 15 nach einem sehr steilen Anstieg 17 in einer Plateauphase, solange die Feuchtebeaufschlagung anhält. Eine entsprechende Auswertung des Verlaufes der Signaldifferenzwerte 15 ergibt, dass sowohl die Steilheit des Anstiegs 17, als auch die Abweichungsdauer, also die Dauer 18 der Signaldifferenzabweichung, die zuvor bestimmten Grenzwerte übersteigt. Daher wird der Signalanstieg 16 nicht einer Gasdetektion zugeordnet, sondern als feuchteinduzierte Störung erkannt. Aus diesem Grund wird der Brandmelder 1, der den Sensor A verwendet, durch die mit der Auswerteeinrichtung 5 verbundene Kontrolleinrichtung 6 zur Vermeidung eines feuchteverursachten Fehlalarms in einen Störungsmodus versetzt. Bei den Sensoren B und C hingegen führt der Feuchteanstieg nicht zu einer Kondensation.

Nach dem Absinken der Feuchte unter einen bestimmen Wert bei etwa t = 900 min sinken die Werte der Signaldifferenz 15 auf den Ausgangswert um Null zurück. Ab diesem Moment reagiert der Sensor A wieder normal auf Gase und die Störmeldung wird wieder aufgehoben, so dass ein normaler Melderbetrieb wieder möglich ist. Um den Zeitpunkt zum Umschalten in einen Normalbetriebsmodus zu ermitteln, erfolgt auch nach dem ersten Umschalten in den Störungsmodus eine weitere Überwachung und Auswertung des Sensorsignals durch die Auswerteeinrichtung 5. Das starke Abfallen des Signals von Sensor A auf einen Wert von etwa -500 mV, und damit weit unter den Normwert um Null, spielt für die weitere Gasdetektion keine Rolle, da dieses Niveau als neues Referenzniveau verwendet werden kann.

Obwohl die Erfindung im Detail durch das bevorzugte Ausführungsbeispiel näher illustriert und beschrieben wurde, so ist die Erfindung nicht auf die offenbarten Beispiele eingeschränkt und andere Variationen können vom Fachmann hieraus abgeleitet werden, ohne den in den Ansprüchen definierten Schutzumfang der Erfindung zu verlassen.

### Bezugszeichenliste

- 1: Brandmelder
- 2: Gassensor
- 3: Meldeeinheit
- 4: SGFET
- 5: Auswerteeinrichtung
- 6: Kontrolleinrichtung
- 7: Spannungsquelle
- 8: Source
- 9: Drain
- 10: Luftspalt
- 11: gassensitive Schicht
- 12: zu detektierendes Gas
- 13: Spannung, Sensorsignal
- 14: zusätzlich eingeprägte Spannung
- 15: Signaldifferenz
- 16: Signalanstieg
- 17: Signaldifferenzanstieg
- 18: Dauer der Signaldifferenzabweichung
- 19: Spannungspuls im Sensorsignal
- 20: Auswertebereich konstanter eingeprägter Spannung
- 21: Pulsdauer
- 22: Feuchteanstieg

## Patentansprüche

1. Verfahren zur Erhöhung der Fehlalarmsicherheit eines Brandmelders (1), der einen Brandgassensor (2) aufweist, welcher einen gassensitiven Feldeffekttransistor (4) umfasst, wobei an einem Gate des Feldeffekttransistors (4) eine zusätzliche Potentialänderung (14) eingeprägt wird, die eine Änderung des Sensorsignals (13) zur Folge hat, wobei wenigstens eine feuchteabhängige Größe dieser Signaländerung ausgewertet und der Brandmelder (1) zur Vermeidung eines feuchteverursachten Fehlalarms in einen Störungsmodus versetzt wird, wenn ein bestimmtes Auswerteergebnis vorliegt.

2. Verfahren nach Anspruch 1, **dadurch gekennzeichnet,**
**dass** die Differenz (15) zwischen dem Sensorsignal (13) zu einem ersten Zeitpunkt und dem Sensorsignal (13) zu einem zweiten Zeitpunkt während der zusätzlichen Potentialänderung (14) ausgewertet wird.

3. Verfahren nach Anspruch 2, **dadurch gekennzeichnet,**
**dass** die Änderung (17) des Signaldifferenzwertes (15) ausgewertet wird.

4. Verfahren nach Anspruch 2 oder 3, **dadurch gekennzeichnet, dass** die Dauer (18) der Signaländerung ausgewertet wird.

5. Verfahren nach einem der Ansprüche 1 bis 4,
**dadurch gekennzeichnet, dass** der Verlauf des Sensorsignals (13) zwischen einem ersten Zeitpunkt und einem zweiten Zeitpunkt während der zusätzlichen Potentialänderung (14) ausgewertet wird.

6. Verfahren nach einem der Ansprüche 1 bis 5,
**dadurch gekennzeichnet, dass** der erste Zeitpunkt zu Beginn der zusätzlichen Potentialänderung (14) und der zweite Zeitpunkt am Ende der zusätzlichen Potentialänderung (14) liegt.

7. Verfahren nach einem der Ansprüche 1 bis 6,
**dadurch gekennzeichnet, dass** die zusätzliche Potentialänderung (14) in Form eines Spannungsimpulses eingeprägt wird.

8. Verfahren nach einem der Ansprüche 1 bis 7,
**dadurch gekennzeichnet, dass** das Einprägen der zusätzlichen Potentialänderung (14) in Abständen wiederholt wird.

9. Brandmelder (1) mit einem Brandgassensor (2), der einen gassensitiven Feldeffekttransistor (4) umfasst, wobei mit Hilfe einer Spannungsquelle (7) an einem Gate des Feldeffekttransistors (4) eine zusätzliche Potentialänderung (14) einprägbar ist, die eine Änderung des Sensorsignals (13) zur Folge hat, mit einer Auswerteeinrichtung (5) zur Auswertung wenigstens einer feuchteabhängigen Größe einer solchen Sensorsignaländerung und einer Kontrolleinrichtung (6), die dazu ausgebildet ist, den Brandmelder (1) zur Vermeidung eines feuchteverursachten Fehlalarms in einen Störungsmodus zu versetzen, wenn ein bestimmtes Auswerteergebnis vorliegt.

## Claims

1. Method for decreasing the possibility of a fire alarm (1) producing a false alarm, comprising a fume sensor (2) having a gassensitive field effect transistor (4), wherein an additional change in potential (14) is applied to a gate of the field effect transistor (4), which results in a change in the sensor signal (13), wherein at least one moisture-dependent variable of this change in signal is evaluated and the fire alarm (1) is moved into interference mode in order to prevent a fire alarm caused by moisture if a specific evaluation result exists.

2. Method according to claim 1, **characterised in that** the difference (15) between the sensor signal (13) at a first point in time and the sensor signal (13) at a second point in time is evaluated during the additional change in potential (14).

3. Method according to claim 2, **characterised in that** the change (17) in signal difference value (15) is evaluated.

4. Method according to claim 2 or 3, **characterised in that** the duration (18) of the change in signal is evaluated.

5. Method according to one of claims 1 to 4,
**characterised in that** the course of the sensor signal (13) between a first point in time and a second point in time is evaluated during the additional change in potential (14).

6. Method according to one of claims 1 to 5,
**characterised in that** the first point in time lies at the start of the additional change in potential (14) and the second point in time lies at the end of the additional change in potential (14).

7. Method according to one of claims 1 to 6,
**characterised in that** the additional change in potential (14) is applied in the form of a voltage pulse.

8. Method according to one of claims 1 to 7,
**characterised in that** the additional change in potential (14) is repeatedly applied at intervals.

9. Fire alarm (1) having a fume sensor (2) with a gas-sensitive field effect transistor (4), wherein with the aid of a voltage source (7), an additional change in potential (14) can be applied to a gate of the field effect transistor (4), which results in a change in the sensor signal (13), having an evaluation facility (5) for evaluating at least one moisture-dependent variable of such a change in sensor signal and a control facility (6) which is embodied to move the fire alarm (1) into interference mode in order to prevent a false alarm caused by moisture if a specific evaluation result exists.

## Revendications

1. Procédé destiné au renforcement de la sécurité contre les fausses alarmes d'un détecteur d'incendie (1) qui présente un détecteur de gaz de combustion (2), lequel comprend un transistor à effet de champ (4) sensible aux gaz, dans lequel au niveau d'une grille du transistor à effet de champ (4) est appliquée une variation de potentiel supplémentaire (14) qui a pour conséquence une variation du signal de détecteur (13), dans lequel au moins une grandeur, dépendant de l'humidité, de cette variation de signal est évaluée et le détecteur d'incendie (1) est mis en mode de dérangement afin d'éviter une fausse alarme due à l'humidité en présence d'un résultat d'évaluation donné.

2. Procédé selon la revendication 1, **caractérisé en ce que** la différence (15) entre le signal de détecteur (13) à un premier moment et le signal de détecteur (13) à un deuxième moment pendant la variation de potentiel supplémentaire (14) est évaluée.

3. Procédé selon la revendication 2, **caractérisé en ce que** la variation (17) de la valeur de différence de signaux (15) est évaluée.

4. Procédé selon la revendication 2 ou 3, **caractérisé en ce que** la durée (18) de la variation de signal est évaluée.

5. Procédé selon l'une des revendications 1 à 4,
**caractérisé en ce que** le comportement du signal de détecteur (13) entre un premier moment et un deuxième moment pendant la variation de potentiel supplémentaire (14) est évalué.

6. Procédé selon l'une des revendications 1 à 5,
**caractérisé en ce que** le premier moment est au début de la variation de potentiel supplémentaire (14) et le deuxième moment est à la fin de la variation de potentiel supplémentaire (14).

7. Procédé selon l'une des revendications 1 à 6,
**caractérisé en ce que** la variation de potentiel supplémentaire (14) est appliquée sous la forme d'une impulsion de tension.

8. Procédé selon l'une des revendications 1 à 7,
**caractérisé en ce que** l'application de la variation de potentiel supplémentaire (14) est répétée à intervalles.

9. Détecteur d'incendie (1) comportant un détecteur de gaz de combustion (2), lequel comprend un transistor à effet de champ (4) sensible aux gaz, dans lequel à l'aide d'une source de tension (7) une variation de potentiel supplémentaire (14) est applicable au niveau d'une grille du transistor à effet de champ (4), laquelle variation a pour conséquence une variation du signal de détecteur (13), comportant un dispositif d'évaluation (5) destiné à l'évaluation d'au moins une grandeur, dépendant de l'humidité, d'une telle variation de signal de détecteur et un dispositif de commande (6) qui est conçu pour mettre le détecteur d'incendie (1) en mode de dérangement afin d'éviter une fausse alarme due à l'humidité en présence d'un résultat d'évaluation donné.
